# EUROPEAN PATENT SPECIFICATION

(11) **EP 4 316 468 B1**
(45) Date of publication and mention of the grant of the patent: **25.12.2024**
(21) Application number: 23189118.5
(22) Date of filing: 02.08.2023
(51) Int. Cl.: A61K 9/14

(54) **INNOVATIVE COMPLEXATION OF LIPIDO-STEROLIC SERENOA EXTRACT WITH TADALAFIL**
INNOVATIVE KOMPLEXIERUNG EINES LIPID-STEROLISCHEN SERENOA-EXTRAKTS MIT TADALAFIL
COMPLEXATION INNOVANTE D'EXTRAIT DE SERENOA LIPIDO-STÉROL AVEC DU TADALAFIL

(30) Priority: 03.08.2022 IT 202200016533
(43) Date of publication of application: 07.02.2024
(73) Proprietor: Recordati Industria Chimica E Farmaceutica SPA, 20148 Milano (IT)
(72) Inventor: MONETTI, Vincenzo, I-06012 Città di Castello (PG) (IT)
(74) Representative: Dragotti & Associati S.R.L.

(56) References cited:
- WO-A2-2011/030351
- US-A1- 2008 261 995
- MOHAMMED M. MEHANNA, ADEL M. MOTAWAA, MAGDA W. SAMAHA: "Tadalafil Inclusion in Microporous Silica as Effective Dissolution Enhancer: Optimization of Loading Procedure and Molecular State Characterization", JOURNAL OF PHARMACEUTICAL SCIENCES, vol. 100, no. 5, 1 May 2011 (2011-05-01), pages 1805 - 1818, XP009542882

## Description

The present invention relates to a method for making capsules or tablets containing Serenoa repens oil in effective and stable association with Tadalafil, for the treatment and prevention of benign prostatic hypertrophy.

More specifically, the method involves the adsorption of Serenoa oil onto a solid excipient consisting of mesoporous silica according to a specific operating procedure which allows the preparation of a solid pharmaceutical form (tablets or opercolated capsules) in which two active and pharmacologically tested ingredients, such as Serenoa oil and Taladafil naturally present in a different physical state, are coupled.

### - Benign prostatic hypertrophy

Prostate volume enlargement, known as benign prostatic hypertrophy, is due to the proliferation of the cells of the stroma and the glandular component of the central part of the gland.

It is a particularly frequent pathology in the adult-elderly: 80% of men over 55 years of age are affected, even if it clinically manifests itself only in a low percentage of subjects, depending on the resulting degree of obstruction.

The main cause is a hormonal dysregulation in the prostate tissue, where dihydrotestosterone stimulates cell reproduction. Responsible for the increase in volume of the gland, however, is also a certain degree of tissue edema. Testosterone, transported into the circulation by a specific protein, is released into the prostate cells where it is transformed into its active form, dihydrotestosterone, by the enzyme 5 alpha reductase.

The enzyme 5-alpha reductase is highly concentrated in the skin, liver, central nervous system (where the type 1 isoform is predominantly present) and prostate (where the type 2 isoform is concentrated).

If produced in excess, by virtue of its strong androgenic power, dihydrotestosterone favors the appearance of acne, accelerates hair loss, and causes prostatic hypertrophy.

Hence the use of drugs of phytotherapeutic (such as Serenoa) or synthetic (such as finasteride) derivation, capable of blocking the enzyme 5-alpha-reductase in the treatment of these conditions.

However, unlike finasteride and dutasteride, which inhibit only one or both isoforms of the 5α-reductase enzyme, Serenoa acts at a dual level, blocking the enzyme on the one hand, and preventing the interaction of DHT with its receptor on the other.

Equally significant and known is the use of Taladafil which, by inhibiting phosphodiesterase 5 which is widely distributed in the corpora cavernosa, contributes to the decrease in the proliferation of smooth muscle cells, potentially reducing the prostate size.

The complexation within a single solid pharmaceutical formulation of Serenoa repens oil with Tadalafil, which is a crystalline powder at room temperature, would therefore be highly desirable.

Let us now compare the characteristics of these two active ingredients:

### - Tadalafil

It is a selective inhibitor of phosphodiesterase 5 which is widely distributed in the corpora cavernosa.

PDE-5 inhibition leads to an accumulation of cGMP and therefore has a pro-erective effect. It has binding affinity for PDE-5 which is five times greater than Sildenafil.

It is used for the treatment of erectile dysfunction, pulmonary hypertension, and benign prostatic hypertrophy.

In BPH, tadalafil may contribute to decrease smooth muscle cell proliferation, potentially reducing prostate size and relieving the anatomical obstruction that produces BPH.

Tadalafil has a MW of 389.4; XLogP3-Aa: 2.3; pKa:0.85;
The molecule is practically insoluble in water, 220mg/L at 25 °C.

The molecule results to be well absorbed following oral administration. Unlike Sildenafil and Vardenafil, the absorption rate of Tadalafil is not affected by the presence or absence of food in the stomach.

Tadalafil has a Tmax of 0.5-6 hours with a median of two hours in healthy adults.

It is mainly eliminated through hepatic metabolism.

The metabolization is accomplished by CYP3A4, the main metabolite is poorly active and is eliminated to a greater extent in the feces (61%) and partly in the urine. However, the pharmacokinetic characteristic that distinguishes Tadalafil is its half-life of approximately 17 hours, which makes it easier to take the drug compared to Sildenafil and Vardenafil.

The intake of tadalafil by those patients who are under treatment with nitroderivatives is specifically contraindicated. Adequate precautions should also be taken in subjects treated with antihypertensive drugs.

The most common side effects are called "flushing" and are headache, nasal congestion, hypotension.

The Food and Drug Administration has approved the updated labeling for Cialis to reflect a small number of post-marketing reports of sudden vision loss attributed to NAION (non-arteritic ischemic optic neuropathy), a condition in which blood flow to the optic nerve is blocked. The FDA advises patients to stop taking these medicines and call a doctor or healthcare professional right away if they experience sudden or decreased vision loss in one or both eyes. Compared to Sildenafil and Verapamil the reduced affinity of tadalafil for PDE6 may explain the reduced incidence of visual side effects.

The maximum recommended dose is 20 mg.

### - Serenoa Repens

Serenoa (Serenoa repens = Sabal serrulata - Serenoa serrulata) is the name of a palm that grows in the states bordering the southern Atlantic coast of the United States, in southern Europe and northern Africa. This small palm (not exceeding 3 meters in height) produces dark red, single-seeded berries similar in size to olives, which form the drug.

Serenoa Repens or Sabal serulata has a selective antiandrogenic action on the prostate and an anti-edema action, without inhibiting the pituitary gland and without acting on estrogen and progesterone.

In fact, it works by inhibiting two enzymes, 5-alpha reductase, the enzyme responsible for the transformation of testosterone into dihydrotestosterone, and aromatase, the enzyme that leads to the formation of 17-beta-estradiol from testosterone.

Pharmacological research has also made it possible to study the complex mechanism of action of Serenoa:
1- Inhibition of 5 alpha reductase (Hagenlocher, 1993; Bayne 200);
2- Blockade of cyclooxygenase and lipoxygenase activity (Breu, 1992);
3- Inhibition of leukotriene synthesis (Paubert-Braquet, 1997);
4- Attenuation of the proliferative response to growth factors (Paubert-Braquet, 1998);
5- Inhibitory effect on nuclear estrogen receptors (Di Silverio; 1992);
6- Interference with prolactin activity (Vacher, 1995);
7- Antagonist activity on alpha-adrenergic receptors (Odenthal,1996);
Numerous RCTs confirm the efficacy and safety of Serenoa repens in the treatment of benign prostatic hypertrophy.

A systematic literature review by The Cochrane Collaboration (Wilt, 2002) has highlighted 21 RCTs carried out with Serenoa confirming its efficacy; a second more recent meta-analysis (Boyle, 2004) examined RCTs involving a total of 4280 patients which showed that the phytotherapeutic extract reduces the IPSS (International Prostate Symptom Score) by about five points, with an improvement in urinary flow and nocturia.

The trials in which Serenoa was also tested against finasteride, the reference drug which inhibits 5 alpha reductase (Fong, 2005; Wilt, 2002; Strauch, 1994) and against tamsulosin, the alpha inhibitor reference drug (Debruyene, 2004) are significant, demonstrating equal efficacy, alone or in association with a nettle extract (Lopatkin, 2007; Hizli, 2007; Engelmann, 2006; Popa, 2005).

The RCTs show a high safety profile for Serenoa: there are rare side effects such as epigastric pain, nausea, and dyspepsia; a case of acute hepatitis, a case of acute pancreatitis in an alcoholic patient, and an increase in bleeding in the postoperative period are described.

Serenoa oil consists mainly of free fatty acids (oleic, lauric, palmitic, cis-linoleic and myristic acids) and phytosterols (Beta sitosterol, campesterol, stigmasterol).

The literature confirms that the treatment with this extract provides for: 320 mg/dose of lipido-sterolic extract, 2 times a day after meals; prolonged treatments lasting not less than six months, to be repeated periodically. WO 2011/030351 discloses a method for making capsules or tablets containing Serenoa Repens oil in effective and stable association with Tadalafil, for the treatment and prevention of erectile dysfunction.

### Healing properties against benign prostatic hyperplasia

Serenoa repens is now widely used in the treatment of benign prostatic hypertrophy and androgenic alopecia (hair loss caused by endocrine disorders). Its active ingredients (triglycerides, phytosterols, sitosterol derivatives, flavonoids) give it antiandrogenic properties, which are carried out mainly through a direct action on dihydrotestosterone receptors, and indirectly through the inhibition of the 5-alpha-reductase enzyme.

Compositions containing Tadalafil and Serenoa Repens for the treatment of benign prostatic hyperplasia have long been known.

From a prior art search carried out by the Applicant, numerous patent documents were found, among which the following can be mentioned:
1) Patent publication US20190183952 A1 entitled "Methods for preparing active extract and application thereof", which describes a pharmaceutical composition comprising TADALAFIL, saw palmetto and the condition and/or disease selected from the group consisting of a symptom of the lower urinary tract due to benign hypertrophic prostate is described.
2) Publication US20070093493 A1 entitled "Treatment of benign prostatic hypertrophy and lower urinary tract symptoms", which describes a method for treating benign prostatic hypertrophy (BPH) by means of a compound (I) to which the name of TADALAFIL is given, and where serrated palmetto, which is analogous to Serenoa repens, is mentioned as a possible second therapeutic agent to be administered concomitantly with said compound (I).

This patent document is significant in that it highlights the fact that the first and second therapeutic agents are not coupled in a single solid pharmaceutical form, but a kit is provided which comprises a first container containing a composition containing from about 1 mg to about 20 mg of Compound (I), an optional second container containing a composition comprising a second therapeutic drug capable of treating BPH or LUTS, and an insert package providing for chronic, *e.g.* daily administration, of Compound (I) for the treatment of an individual suffering from BPH or LUTS.

In another embodiment, the first container contains a composition comprising from about 1 mg to about 20 mg of Compound (I) and a second therapeutic drug capable of treating BPH or LUTS.

3) The patent publication number WO2007072156 A1 entitled "Pharmaceutical combination of a Pde-5 inhibitor and a 5-alpha reductase inhibitor - Combinaison Pharmaceutique D'Un Inhibiteur De Pde-5 Et D'Un Inhibiteur De 5-Alpha Reductase" which describes a pharmaceutical formulation comprising TADALAFIL, and Serenoa repens is cited for the treatment of LUTS associated with BPH.

4) Patent publication number US20070155837 A1 entitled "Enhancement Of Urogenital Function", which refers to a composition comprising TADALAFIL, saw palmetto (Serenoa repens analogue supplement) for administration to patients diagnosed with BPH (analog of benign prostatic hyperplasia.

5) Patent publication number WO2018204764 A1 entitled "Identification And Targeted Modulation Of Gene Signaling Networks" which describes the perturbative stimulus formulated as a pharmaceutical composition containing TADALAFIL and Serenoa repens which can be used in the treatment of benign prostatic hyperplasia.

From the data found, it is evident that the use of Tadalafil and Serenoa repens in combination, for the treatment of benign prostatic hypertrophy, is certainly not new.

However, in the light of the different physical state characterizing the two active ingredients at room temperature, which in itself represents an obstacle to their complexation within a single solid pharmaceutical formulation, no teaching is provided or suggested by prior art documents to overcome this problem, in order to allow the administration of Serenoa oil in association with Taladafil in the form of tablets or opercolated capsules, avoiding the use of soft-gels.

A first object of the present invention is therefore the development of a complexation method aimed at enhancing the association between said active ingredients, overcoming the obstacle due to the different physical state in which said active ingredients are naturally found.

Another object of the invention is to develop a formulation of a stable and effective pharmaceutical form which, through a careful selection of suitable excipients necessary to facilitate administration and compatible with the indicated active ingredients, by promoting a reproducible release and an adequate bioavailability of the same active ingredient, protects it at the same time from degradation.

Another object of the present invention is to define a drug-loading process based on the adsorption properties of a mesoporous material, such as mesoporous silicas, hydrophilic powders with spherical granules with an average diameter of less than 50 microns, whose most important characteristic is that they can adsorb large amounts of liquids, thanks to the volume of the mesopores, thus acting as an excellent carrier.

This makes them suitable for formulating liquisolid systems with drugs which are liquid at room temperatures, are low melting or are dissolved, dispersed or emulsified.

According to the invention, the adsorption of Serenoa oil onto a solid excipient allows liquid formulations to be administered in the form of tablets or opercolated capsules containing a formulation of Tadalafil + Serenoa oily extract, capable of enhancing the synergistic action of the Tadalafil/Serenoa combination, avoiding the use of soft-gels.

The first technical obstacle to overcome is, in fact, the

### Physical state of the actives

Tadalafil is in the solid state at room temperature, in the form of a white crystalline powder.

Serenoa repens oil, on the other hand, is in an oily liquid form.

The different physical state charactering the two active ingredients at room temperature is in itself an obstacle to their complexation within a single solid pharmaceutical formulation.

For this reason, the Applicant selected suitable excipients compatible with the indicated active ingredients, and a complexation method aimed at enhancing the association was developed.

The adsorption of Serenoa oil onto a solid excipient allows the administration of liquid formulations in the form of tablets or opercolated capsules, thus avoiding the use of soft-gels.

Thanks to the use of this technique, Serenoa Repens oil can be used in association with Tadalafil and capsules can be made.

Further characteristics and advantages of the invention will become apparent from the following detailed description, which illustrates the operating procedure followed by the inventors and the experimental results obtained, with reference to the enclosed table of drawings in which:

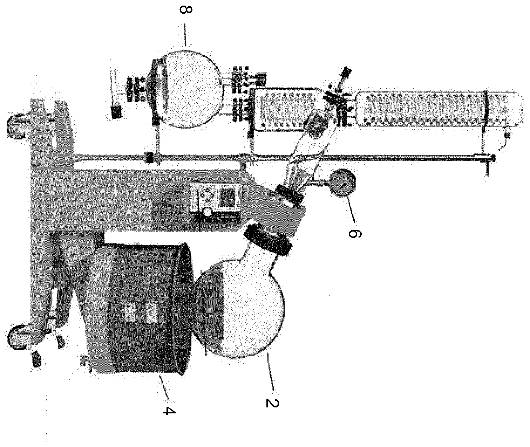

Figure 1 shows a rotary evaporator used for the removal of the solvent from the various samples prepared.

### DETAILED DESCRIPTION OF THE INVENTION

The first operational step consisted in:

### SELECTION OF EXCIPIENTS

Three possible excipients suitable for their adsorbent characteristics were examined: Colloidal Anhydrous Silica, Pregelatinized Starch, and Mesoporous Silica. **1) Colloidal Anhydrous Silica** (Synonyms: Aerosil, R972, silica dimethylsilylate).

### Characteristics

Hydrophobic colloidal silica has nanometer-sized primary particles and a large specific surface area, which provide desirable flow characteristics to dry powders used in tablet and capsule filling. Hydrophobic grades allow for lower moisture absorption; this may offer an advantage in moisture sensitive formulations.

Hydrophobic colloidal silica is also used to thixotropically control viscosity, to thicken and stabilize emulsions or as a suspending agent in gels and semi-solid preparations. Hydrophobic colloidal silica has a less pronounced effect on solution viscosity but can thicken and stabilize the oil phase of a water-oil emulsion. It has good absorbent properties.

Clear gels can be formed with other ingredients of similar refractive index.

In general, uses and ranges of hydrophobic colloidal silica are similar to the used concentrations of standard hydrophilic colloidal silicon dioxide.

Hydrophobic colloidal silica is prepared by flame hydrolysis of chlorosilanes, such as silicon tetrachloride, at 1800°Ct using a hydrogen-oxygen flame. It is rapidly cooled to create an amorphous product and immediately treated with dichlorodimethylsilane in a fluid bed reactor.

The resulting surface is covered with dimethylsilyl groups.

In the experimental tests carried out, AEROSIL^{®} R 972 was used; it is a silica-based rheological additive produced by EVONIK Operations Gmbh by modifying the surface of another previous product, Aerosil 130, and therefore has primary particles of the same size and same surface area, but the density of silanol groups is reduced from about 2.0 SiOH/nm² to 0.75 SiOH/nm².

The term colloidal indicates a characteristic state of aggregation distinct from solutions and suspensions: a substance is in this state when it is dispersed in another substance in the form of generally amorphous particles.

### Safety:

The safety profile of hydrophobic colloidal silica is the same as hydrophilic types of silica, since the silica modified surface does not significantly alter the toxicological properties.

LD50 (rat, IV): 0.015 g/kg

### Regulatory:

It is approved for use in pharmaceuticals in Europe. It is approved by the FDA and in Europe for food contact items. It is included in non-parenteral medicines (oral tablets; rectal suppositories) authorized in the UK.

### 2) Pregelatinized starch

### Preparation

Pregelatinized starches for food use are prepared by heating an aqueous suspension containing up to 42% w/w starch to 62-728°C.

Chemical additives that may be included in the slurry are gelatinization aids and surfactants, added to control rehydration or minimize sticking during drying.

After heating, the slurry can be spray dried, roller dried, extruded or drum dried.

In the latter case, the dried material can be processed to produce a desired particle size range.

Fully pregelatinized starch pharmaceutical grades use no additives and are prepared by spreading an aqueous suspension of ungelatinized starch over hot drums where gelatinization and subsequent drying take place.

Partially pregelatinized starch is produced by subjecting moistened starch to mechanical pressure. The resulting material is ground, and the moisture content adjusted to specification.

Pregelatinized starch is starch that has been chemically and mechanically processed to fully or partially break down the starch granules. Both fully and partially pregelatinized grades are commercially available. The partial pre-gelatinization makes the starch flowable and directly compressible.

Full pregelatinization produces a starch soluble in cold water that can be used as a binder in wet granulation. Typically, pregelatinized starch contains 5% free amylose, 15% free amylopectin, and 80% unmodified starch. USP32-NF27 does not specify the botanical origin of the original starch, but PhEur 6.3 specifies that pregelatinized starch is obtained from corn (maize), potato or rice starch. Fully pregelatinized starch typically contains 20-30% amylose with the remainder being amylopectin, which is roughly the same ratio (1:3) as the partially pregelatinized form. There are ways to increase the amylose portion.

### Characteristics:

Partially pregelatinized starch is a modified starch used in oral capsule and tablet formulations as a binder, diluent, and disintegrant.

Compared to starch, partially pregelatinized starch can be produced with improved flow and compression characteristics, so that the pregelatinized material can be used as a tablet binder in dry compression or direct compression processes. In such processes, the pregelatinized starch is self-lubricating. However, when used with other excipients it may be necessary to add a lubricant to a formulation. Although 0.25% w/w magnesium stearate is commonly used for this purpose, concentrations higher than this may have adverse effects on tablet strength and dissolution. Thus, stearic acid is generally the preferred lubricant with pregelatinized starch.

It also has good absorbent properties.

Partially pregelatinized starch is used in oral dry powder hard capsule formulations.

Both partially and fully pregelatinized starch can also be used in wet granulation processes.

Fully pregelatinized starches can be used to make soft capsules, shells, and coatings, as well as binders in tablets.

### Safety:

Starch and pregelatinized starch are widely used in oral solid dosage formulations.

Pregelatinized starch is generally considered to be a non-toxic and non-irritating excipient.

### Regulatory:

It is included in the FDA's Inactive Ingredients Database (oral capsules, suspensions, and tablets; vaginal preparations). It is included in non-parenteral medicines authorized in the UK.

### 3) Mesoporous silica

### Synonyms: Aeroperl 300, Syloid

Present in nature in various forms (crystalline, amorphous, impure), this solid is now available with very peculiar micro-structures developed *ad hoc* for specific technological applications. Pharma-grade silica is invariably amorphous (the crystalline one would involve the risk of silicosis) and can be traced back to two main types: pyrogenic and precipitated.

The first is obtained by flame hydrolysis of SiCl₄ in an atmosphere of hydrogen and oxygen at high temperature (hence the denomination of pyrogenic); the second by precipitation with sulfuric acid from solutions of sodium silicate.

Pyrogenic silica is highly pure (the main impurity is HCl in concentrations lower than 0.025%) and is formed by aggregates of primary particles which link together thus creating an inter-particle porosity. The precipitated one may contain water residues up to 7-8% and traces of alkali and alkaline-earth metals; compared to pyrogenic silica, in origin, it has a higher apparent density and a porosity developed also at an intra-particle level.

In both cases, by modifying the production conditions, products with different chemical-physical and structural characteristics are obtained. As a consequence, the following may change: specific surface area, volume of meso-pores, compaction density, flowability of the powder, adsorption capacity, gelling capacity, and various other parameters.

In the pharmaceutical use, mesoporous granular silica, which guarantees superior technical performance and concrete operational advantages, has taken over; Aeroperl300^{®} (by Evonik, Germany) and Syloid^{®} (by Grace, USA) are among the most popular products on the market. The former is fumed silica, while the latter are precipitated silica gels.

These are hydrophilic powders with spherical granules having an average diameter of less than 50 microns, exceptionally free-flowing (rest angle of about 35°) and high apparent density. The most interesting feature is that, thanks to the volume of the mesopores, they can adsorb large amounts of liquids (up to 1.5:1-liquid: silica) acting as excellent carriers.

This makes them suitable for formulating liquisolid systems with drugs which are liquid at room temperatures, are low melting or are dissolved, dispersed or emulsified.

Mesoporous silicas are characterized by:
- Porosity of homogeneous size which allow to control drug loading and release kinetics.
- A high porous volume which allows to encapsulate a good amount of active ingredient.
- A high specific surface area, which promotes drug adsorption.

Pore size is considered to be one of the most important properties of mesoporous materials.

It determines the size of the molecules that can be adsorbed in the mesopores. Generally, if the pore diameter is slightly larger than the size of the drug molecule, and therefore if the pore/molecule size ratio is greater than 1, the molecule is adsorbed inside the mesopores.

Furthermore, an important characteristic of mesoporous materials is that the diameter of the mesopores can be varied from 1.5 nm to tens of nm either by changing the length of the surfactant chains or by solubilizing auxiliary substances in the micelles. In this way, therefore, not only simple molecules but also macromolecules can be adsorbed.

### Pharmaceutical Development

After examining three possible excipients suitable for their adsorbent characteristics, some preliminary reasoning and tests were carried out.

The success of the formulation of a stable and effective pharmaceutical form depends on a careful choice of excipients necessary to facilitate the administration, promoting a consistent release and adequate bioavailability of the active ingredient, also protecting it from degradation.

Compatibility studies concern drug interactions with various excipients, but drug-drug interactions in therapeutic associations also deserve attention.

The drug-loading process is based on the adsorption properties of the mesoporous material.

The surface has a fundamental role in the amount of active ingredient adsorbed inside the silica.

Silica surface has therefore been the object of numerous studies. In 1934 Hofman hypothesized the existence of silanol groups (SI-OH) on silica surface. Since then, numerous scientists have worked on this topic and confirmed the existence of these groups through analytical techniques.

In particular, the following can be distinguished: single, vicinal, germinal, and internal silanols.

The isolated silanols are free to form hydrogen bonds with the adsorbates, in our case the molecules of Tadalafil and Serenoa repens.

Increase in the temperature results in the disappearance of various silanols groups, therefore by treating silica at high temperatures its hydrophobic character is increased to the detriment of the hydrophilic character.

In silicas treated at high temperatures (colloidal anhydrous), in fact, the adsorption process is dominated by hydrophobic Van der Waals interactions, mainly dispersive forces.

The preparation method also affects the stability and quality of the prepared drug.

There are numerous methods of incorporation and adsorption of the active ingredients in the carrier.

In the experimental tests carried out, a Rotavapor as illustrated in Fig. 1 was used.

### Complexation by Rotavapor or Rotary Evaporator

The rotary evaporator or rotavapor is an instrument used for the removal of a solvent from a sample.

In our specific case, three different samples were prepared:
1- Colloidal anhydrous silica/Tadalafil
2- Pregelatinized Corn Starch/Tadalafil
3- Mesoporous silica Aeroperl 300/Tadalafil/

With reference to Fig. 1, the instrument illustrated by way of non-limiting example only consists of a rotating flask 2 into which the solvent solution and the sample are placed: it is placed into a bath 4 of hot water and rotated. A vacuum system equipped with a vacuum gauge 6, by reducing the pressure in the apparatus, causes a decrease in the boiling temperature of the solvent. With this method the evaporation rate of the solvent is much higher.

The evaporated solvent is made to condense in a second flask 8, and once the evaporation is complete, the drug is incorporated in the carrier and the powder can be recovered.

### Processing Method

### Method of preparation:

In all three samplings it was decided to prepare 100 capsules with a content per capsule of:
**Taladafil: 5 mg**
**Lipido-sterolic Serenoa extract,** at 85-95% in free fatty acids (oleic, lauric, palmitic, cis-linoleic, myristic acids) and phytosterols (Betasistosterol, campesterol, stigmasterol): **200 mg.**

To make 100 capsules, it was therefore decided to initially weigh 500 mg of Tadalafil in all three experiments.

### Experiment with colloidal anhydrous silica

After weighing 500 mg of Tadalafil, 20 g of Colloidal Anhydrous Silica, Aerosil R972, were weighed.

The calculation is based on the amount of 200 mg: this suitable amount was chosen for multiple factors.
A) The documented adsorbent power of colloidal anhydrous silica capable of adsorbing oils for 80-120% its weight.
   In this specific case, therefore, a previous adsorption test was carried out, which was fully completed with 300 mg of Colloidal Anhydrous Silica perfectly capable of adsorbing 320 mg of lipido-sterolic Serenoa extract.
B) The size of the colloidal anhydrous silica micropores is optimal for the incorporation of Tadalafil.

Tadalafil and Aerosil R972 were placed inside the Rotavapor flask. Then, 400 to 800 ml of ethanol were added into the same flask. The solution containing the powders and the solvent was sonicated for a period of time ranging from 100 to 300 seconds, and finally the rotating flask 2 was connected to the rotavapor for a period of time ranging from 30 to 50 minutes at a temperature ranging from 25 to 50 °C, in order to evaporate the solvent and obtain the sample of silica impregnated with Tadalafil. It was decided to apply pressure ranges from 1 to 10-3 mbar.

Subsequently, Serenoa oil was incorporated with the aid of a knife mill, adding the oil at 5 ml per step.

After each single addition, the knife mill was operated for a period of time ranging from 10 to 60 seconds.

The obtained powder was sieved with a 140 mesh sieve and mixed.

Before preparing one hundred capsules, the powder was placed in a cylinder for calculating the apparent volume. Unfortunately, the Volume turned out to be of 180 ml and it was therefore impossible to prepare the capsules for volumetric reasons.

Even with a "triple zero" capsule type, the volume required to prepare 100 capsules would have been approximately 108 ml.

The experiment showed one of the negative characteristics of this type of silica compared to mesoporous granular silica.

The excessive volume occupied by this type of silica did not allow the preparation of the specific pharmaceutical form.

### THE RESULT IS NEGATIVE.

### Experiment with Pregelatinized Starch

After weighing 500 mg of Tadalafil, 30g of pregelatinized corn starch were weighed.

An adsorption test between pregelatinized starch and lipido-sterolic Serenoa extract was performed.

Despite its adsorbent characteristics being documented, starch demonstrated an insufficient adsorption capacity for the lipid-sterolic Serenoa extract.

Its adsorbent properties therefore did not prove to be sufficient and part of the lipido-sterolic Serenoa extract remained in liquid form.

### THE RESULT IS NEGATIVE.

### Experiment with mesoporous granular silica Aeroperl 300

After weighing 500 mg of Tadalafil, 20 g of Aeroperl 300 were weighed.

Tadalafil and Aeroperl 300 were placed inside the Rotavapor flask. Then, 400 to 1600 ml of ethanol were added into the same flask.

The solution containing the powders and the solvent was sonicated for a period of time ranging from 60 to 300 seconds, and finally the rotating flask was connected to the rotavapor for a period of time ranging from 30 to 60 minutes, at a temperature ranging from 25 to 50 degrees °C, in order to evaporate the solvent and obtain the sample of Aeroperl 300 impregnated with Tadalafil. It was decided to apply Pressure ranges from 1 to 10⁻³ mbar.

Subsequently, Serenoa oil was incorporated with the aid of a knife mill, adding the oil at 5 ml per step.

After each single addition, the knife mill was operated for a period of time ranging from 10 to 30 seconds.

The obtained powder was sieved with a 140 mesh sieve and mixed.

Before preparing one hundred capsules, the powder was placed in a cylinder for calculating the apparent volume.

The apparent volume was found to be of 95 ml, therefore suitable for preparing 100 "double zero" type capsules.

The Aeroperl 300 mesoporous granular silica coupled to a specific operating procedure has therefore enabled the preparation of a solid pharmaceutical form in which two active ingredients, naturally present in a different physical state, are coupled.

### Conclusions

From the tests carried out, it was found that:
The adsorption of Serenoa oil onto a solid excipient allows the administration of liquid formulations in the form of tablets or opercolated capsules, avoiding the use of soft-gels;
The adsorption inside the meso-pores of a granular silica exposes the poorly soluble drug to a high exchange surface with the solvent medium, and significantly increases its dissolution rate.

Therefore, thanks to the use of this technique, it is possible to use Serenoa Repens oil in association with Tadalafil and make capsules.

By comparing the chemical characteristics of Tadalafil, and in particular its solubility, it appears clear that this is the only way that allows an effective association in a solid formulation.

The development of a specific mesoporous granular silica with this preparation technique leads to a specific drug carrier able to deliver the drug to the site of interest, thus reducing potential adverse side effects, and potentially allowing a further reduction in the administered dosage.

### BIBLIOGRAPHY

1. Matthias J, Wannemaker G. Basic characteristics and applications of Aerosil 30. The chemistry and physics of the Aerosil surface. J Colloid Interface Sci 1998; 125: 61-68.
2. Zimmermann I et al. Nanomaterials as flow regulators in dry powders. Z Phys Chem 2004; 218: 51-102.
3. Jonat S et al. Investigation of compacted hydrophilic and hydrophobic colloidal silicon dioxides as glidants for pharmaceutical excipients. Powder Technol 2004; 141: 31-43.
4. Jonat S et al. Influence of compacted hydrophobic and hydrophilic colloidal silicon dioxide on tabletting properties of pharmaceutical excipients. Drug Dev Ind Pharm 2005; 31: 687-696.
5. Evonik Industries. Technical bulletin fine particles No. 11: Basic characteristics of Aerosil fumed silica TB0011-1, 2006.
6.Evonik Industries. Technical literature: Aerosil colloidal silicon dioxide for pharmaceuticals TI1281-1, 2006.
7.Waddell WW, ed. Silica Amorphous.Kirk-Othmer Encycopedia of Chemical Technology, 5th edn, vol. 22: New York: Wiley, 2001; 8 -Lewinson J et al. Characterization and toxicological behavior of synthetic amorphous hydrophobic silica. Regul Toxicol Pharmacol
   1994; 20(1): 37-57.
8. Carbone D et al. Process for cooking/drying high-amylose starches. United States Patent 7,118,764; 2002.
9. Small LE, Augsburger LL. Aspects of the lubrication requirements for an automatic capsule filling machine. Drug Dev Ind Pharm 1978; 4: 345-372.
10. Mattson S, Nystro ·· m C. Evaluation of critical binder properties affecting the compactability of binary mixtures. Drug Dev Ind Pharm 2001; 27: 181-194.
11. Rudnic EM et al. Evaluations of the mechanism of disintegrant action. Drug Dev Ind Pharm 1982; 8: 87-109.
12. Manudhane KS et al. Tableting properties of a directly compressible starch. J Pharm Sci 1969; 58: 616-620.
13. Underwood TW, Cadwallader DE. Influence of various starches on dissolution rate of salicylic acid from tablets. J Pharm Sci 1972; 61: 239-243.
14. Bolhuis GK, Lerk CF. Comparative evaluation of excipients for direct compression. Pharm Weekbl 1973; 108: 469-481.
15. Sakr AM et al. Sta-Rx 1500 starch: a new vehicle for the direct compression of tablets. Arch Pharm Chem (Sci) 1974; 2: 14-24.
16. Schwartz JB et al. Intragranular starch: comparison of starch USP and modified cornstarch. J Pharm Sci 1975; 64: 328-332.
17. Rees JE, Rue PJ. Work required to cause failure of tablets in diametral compression. Drug Dev Ind Pharm 1978; 4: 131-156.
18. Shangraw RF et al. Morphology and functionality in tablet excipients for direct compression: part II. Pharm Technol 1981; 5(10): 44-60.
19. Chilamkurti RW et al. Some studies on compression properties of tablet matrices using a computerized instrumental press. Drug Dev Ind Pharm 1982; 8: 63-86.
20. Malamataris S et al. Moisture sorption and tensile strength of some tableted direct compression excipients. Int J Pharm 1991; 68: 51-60.
21. Iskandarani B et al. Scale-up feasability in high-shear mixers: determination through statistical procedures. Drug Dev Ind Pharm 2001; 27: 651-657.
22. Shiromani PK, Clair J. Statistical comparison of high-shear versus low- shear granulation using a common formulation. Drug Dev Ind Pharm 2000; 26: 357-364.
23. Colorcon. Technical literature: Starch 1500. 1997.
24. Jaiyeoba KT, Spring MS. The granulation of ternary mixtures: the effect of the stability of the excipients. J Pharm Pharmacol 1980; 32: 1-5.
25. Carr RL. Particle behaviour storage and flow. Br Chem Eng 1970; 15: 1541-1549.
26. Callahan JC et al. Equilibrium moisture content of pharmaceutical excipients. Drug Dev Ind Pharm 1982; 8: 355-369.
27. Wurster DE et al. A comparison of the moisture adsorption-desorption properties of corn starch USP, and directly compressible starch. Drug Dev Ind Pharm 1982; 8: 343-354.
28. Health and Safety Executive. EH40/2005: Workplace Exposure Limits. Sudbury: HSE Books, 2005 (updated 2007). http://www.hse.gov.uk/ coshh/table1.pdf (accessed 5 February 2009).
29. Seppic. Technical Literature: Sepistab ST200. 1997.
30. Alebiowu G, Itiola OA. Compression characteristics of native and pregelatinized forms of sorghum, plantain, and corn starches, and the mechanical properties of their tablets. Drug Dev Ind Pharm 2002; 28(6): 663-672.
31. Nianzeng Xing et al; Quercetin inhibits the expression and function of the androgen receptor in LNCaP prostate cancer cells; Carcinogenesis; volume 22 ; issue 3; 2021).
32. Soudeh Ghafouri-Fard et al; Emergin impact of quercetin in the tratment of prostate cancer; ScienceDirect Journal of Biomedicine e Pharmacotherapy, June 2021.
33. Feiya Yang et al; Quercetin in a prostate cancer: Chemotherapeutic and chemopreventive effects, mechanisms and clinical application potential; Oncology Reports; 2015.
34. Jun-Shik Choi et al.; Effects of quercetin on the bioavailability of doxorubicin in rats: role of CYP3A4 and P-gp inhibition by quercetin; Arch Pharm Res.2011 April.
35. Himanshu Rastorgi et al.; Evaluation of inhibitory effects of caffeic acid and quercetin on human liver cytochrome p450 activities; Phytother Res; 2014
36. Violetta Mohos et. Al; Inhibitory Effects of Quercetin and Its Main Methyl, sulfate, and Glucuronic Acid Conjugates on Cytichrome P450 Enzymes, and on OATP, BCRP and MRP2 Transporters; Nutrients; 2020.
37. Xueni Wang et al; Kaempferol inhibits benign prostatic hyperplasia by resisting the action of androgen; European Journal of Pharmacology, September 2021
38. Surech Palle et al; Enhancement of oral bioavailability of rivastigmine with quercetin nanoparticels by inhibiting CYP3A4 and esterases; Pharmacol Rep; 2017.
39. Dongu Lee et al; Flavonoids baicalein and kaempferol reduced inflammation in benign prostate hyperplasia patient-derived cells through regulating mitochondrial respiration and intracellular oxygen species. Korean J. food science and technology (2021)
40. Giuseppe Morgia et al; Serenoa repens + selenium + lycopene vs tadalafil 5 mg for the treatment of lower urinary tract symptoms secondary to benign prostatic obstruction; BJU int. Epub Apri 2018

## Claims

1. A method for making capsules or tablets containing Serenoa Repens oil in effective and stable association with Tadalafil, for the treatment and prevention of benign prostatic hypertrophy, **characterized in that** it involves the adsorption of the Serenoa oil onto a solid excipient consisting of granular mesoporous silica according to a specific operating procedure which allows the preparation of a solid pharmaceutical form - tablets or opercolated capsules- in which two active and pharmacologically tested ingredients, naturally occurring in a different physical state, such as Serenoa oil and Taladafil, are coupled.

2. The method according to the preceding claim, **characterized in that** the Serenoa repens oil is a lipid-sterolic extract of Serenoa at 85-95% in free fatty acids (oleic, lauric, palmitic, cis-linoleic, myristic acids) and phytosterols (Beta-sitosterol, campesterol, stigmasterol).

3. The method according to the preceding claim, **characterized in that** the granular mesoporous silica is in the form of hydrophilic powders with spherical granules having an average diameter of less than 50 microns, exceptionally flowable (rest angle of about 35) .

4. The method according to any one of the preceding claims including the following steps:
a) suitable amounts, from 500 mg to 5 kg, of Tadalafil powder and from 200 g to 20 kg o f a granular mesoporous silica, such as Aeroperl, are placed inside a Rotavapor flask;
b) an amount of solvent from 400 to 1600 ml of ethanol is added to the same flask;
c) the solution containing the powders and solvent is sonicated for a time period ranging from 60 to 300 seconds;
d) the rotating flask is connected to the Rotavapor for a time period ranging from 30 to 60 minutes, in a temperature range of the heating tank ranging from 25 to 50 Celsius degrees, under pressure conditions ranging from 1 to 10⁻³ mbar, so as to evaporate the solvent and obtain the Aeroperl 300 sample impregnated with Tadalafil;
e) Serenoa oil is then incorporated by means of a knife mill, adding the oil at 5 ml per step, operating the knife mill for a time period ranging from 10 to 30 seconds after each single addition,
f) the powder obtained and mixed is sieved with a 140-mesh.

5. The method according to the preceding claim, **characterized in that**:
a) 500 mg of Tadalafil powder and 300 g of a granular mesoporous silica, such as Aeroperl, are placed inside the Rotavapor flask;
b) an amount of solvent equal to 600 ml of ethanol is added to the same flask;
c) the solution containing the powders and solvent is sonicated for 240 seconds;
d) the rotary flask is connected to the Rotavapor for 40 minutes at 40 °C so as to evaporate the solvent and obtain the Aeroperl 300 sample impregnated with Tadalafil;
e) Serenoa oil is then incorporated by means of a knife mill, adding the oil at 5 ml per step, operating the knife mill for 20 seconds after each single addition;
f) the powder obtained and mixed is sieved with a 140-mesh sieve.

6. The method according to the preceding claim, **characterized in that** the apparent volume of the powder obtained was 95 ml, thus suitable for preparing 100 "double zero"-type capsules.

7. The method according to the preceding claim, **characterized in that** each capsule or tablet contains 5 mg of Taladafil and 200 mg of 85-95% lipid-sterol extract of Serenoa repens.

## Patentansprüche

1. Verfahren zur Herstellung von Kapseln oder Tabletten, die Serenoa repens-Öl in wirksamer und stabiler Verbindung mit Tadalafil enthalten, zur Behandlung und Vorbeugung von gutartiger Prostatahypertrophie, **dadurch gekennzeichnet, dass** es Adsorption des Serenoa-Öls an einen festen Hilfsstoff enthält, der aus körnigem mesoporösem Siliciumdioxid besteht. Das spezifische Betriebsverfahren ermöglicht die Herstellung einer festen pharmazeutischen Form - Tabletten oder Operculatae Kapseln -, in der zwei aktive und pharmakologisch getestete Inhaltsstoffe, die in natürlicher Form in einem anderen physikalischen Zustand vorkommen, wie Serenoa-Öl und Tadalafil, verbunden sind.

2. Verfahren nach dem vorstehenden Anspruch, **dadurch gekennzeichnet, dass** das Serenoa repens-Öl ein lipidsterolischer Extrakt von Serenoa zu 85-95 % in freien Fettsäuren (Öl-, Laurin-, Palmitin-, cis-Linolsäure, Myristinsäure) und Phytosterinen (Beta-Sitosterin, Campesterin, Stigmasterin) ist.

3. Verfahren nach dem vorstehenden Anspruch, **dadurch gekennzeichnet, dass** das körnige mesoporöse Siliciumdioxid in Form von hydrophilen Pulvern mit kugelförmigen Körnern mit einem durchschnittlichen Durchmesser von weniger als 50 Mikrometern vorliegt, die außergewöhnlich fließfähig sind (Ruhewinkel von etwa 35).

4. Verfahren nach einem der vorstehenden Ansprüche, umfassend die folgenden Schritte:
a) geeignete Mengen von 500 mg bis 5 kg Tadalafil-Pulver und 200 g bis 20 kg eines körnigen mesoporösen Siliciumdioxids, wie Aeroperl, werden in einen Rotavapor-Kolben gegeben;
b) eine Lösungsmittelmenge von 400 bis 1600 ml Ethanol wird in den gleichen Kolben gegeben;
c) die Lösung, die die Pulver und das Lösungsmittel enthält, wird für einen Zeitraum von 60 bis 300 Sekunden im Ultraschallbad behandelt;
d) der Drehkolben wird für einen Zeitraum von 30 bis 60 Minuten in einem Temperaturbereich des Heizbehälters von 25 bis 50 Grad Celsius unter Druckbedingungen von 1 bis 10-3 mbar mit dem Rotavapor verbunden, um das Lösungsmittel zu verdampfen und die mit Tadalafil getränkte Aeroperl 300-Probe zu erhalten;
e) das Serenoa-Öl wird dann mittels einer Messermühle eingearbeitet, wobei das Öl mit 5 ml pro Schritt zugegeben und die Messermühle nach jeder einzelnen Zugabe für einen Zeitraum von 10 bis 30 Sekunden betrieben wird;
f) das erhaltene und vermischte Pulver wird mit einem 140 mm-Sieb gesiebt.

5. Verfahren nach dem vorstehenden Anspruch, **dadurch gekennzeichnet, dass**
a) 500 mg Tadalafil-Pulver und 300 g eines körnigen mesoporösen Siliciumdioxids, wie Aeroperl, in den Rotavapor-Kolben gegeben werden;
b) eine Lösungsmittelmenge in Höhe von 600 ml Ethanol in denselben Kolben gegeben wird;
c) die Lösung, die die Pulver und das Lösungsmittel enthält, 240 Sekunden lang im Ultraschallbad behandelt wird;
d) der Drehkolben 40 Minuten lang bei 40 °C mit dem Rotavapor verbunden wird, um das Lösungsmittel zu verdampfen und die mit Tadalafil getränkte Aeroperl 300-Probe zu erhalten;
e) das Serenoa-Öl dann mittels einer Messermühle eingearbeitet wird, wobei das Öl mit 5 ml pro Schritt zugegeben und die Messermühle nach jeder einzelnen Zugabe 20 Sekunden lang betrieben wird;
f) das erhaltene und gemischte Pulver mit einem 140 mm-Sieb gesiebt wird.

6. Verfahren nach dem vorstehenden Anspruch, **dadurch gekennzeichnet, dass** das scheinbare Volumen des erhaltenen Pulvers 95 ml betrug und somit für die Herstellung von 100 Stück 00-Kapseln geeignet ist.

7. Verfahren nach dem vorstehenden Anspruch, **dadurch gekennzeichnet, dass** jede Kapsel oder Tablette 5 mg Tadalafil und 200 mg 85-95% Lipid-Sterol-Extrakt von Serenoa repens enthält.

## Revendications

1. Procédé de fabrication de capsules ou de comprimés contenant de l'huile de serenoa Repens en association efficace et stable avec le tadalafil, pour le traitement et la prévention de l'hypertrophie bénigne de la prostate, **caractérisé en ce qu'**il implique l'adsorption de l'huile de serenoa sur un excipient solide constitué de silice mésoporeuse granulaire selon un mode opératoire spécifique qui permet la préparation d'une forme pharmaceutique solide - comprimés ou capsules operculées - dans laquelle deux ingrédients actifs et testés pharmacologiquement, se trouvant naturellement dans un état physique différent, tels que l'huile de serenoa et le tadalafil, sont couplés.

2. Procédé selon la revendication précédente, **caractérisé en ce que** l'huile de serenoa repens est un extrait lipido-stérolique de serenoa à 85-95 % en acides gras libres (acides oléique, laurique, palmitique, cis-linoléique, myristique) et en phytostérols (bêta-sitostérol, campestérol, stigmastérol).

3. Procédé selon la revendication précédente, **caractérisé en ce que** la silice mésoporeuse granulaire est sous forme de poudres hydrophiles avec des granules sphériques ayant un diamètre moyen inférieur à 50 microns, exceptionnellement fluides (angle de repos d'environ 35).

4. Procédé selon l'une quelconque des revendications précédentes comprenant les étapes suivantes :
a) des quantités appropriées, de 500 mg à 5 kg, de poudre de tadalafil et de 200 g à 20 kg d'une silice mésoporeuse granulaire, telle que l'aeroperl, sont placées à l'intérieur d'un flacon rotavapor ;
b) une quantité de solvant de 400 à 1600 ml d'éthanol est ajoutée dans le même flacon ;
c) la solution contenant les poudres et le solvant est soniquée pendant une période de temps allant de 60 à 300 secondes ;
d) le flacon rotatif est connecté au rotavapor pendant une durée allant de 30 à 60 minutes, dans une plage de température du réservoir de chauffage allant de 25 à 50 degrés Celsius, dans des conditions de pression allant de 1 à 10⁻³ mbar, de manière à évaporer le solvant et à obtenir l'échantillon d'aeroperl 300 imprégné de tadalafil ;
e) l'huile de serenoa est ensuite incorporée au moyen d'un moulin à couteaux, en ajoutant l'huile à 5 ml par étape, en faisant fonctionner le moulin à couteaux pendant une période allant de 10 à 30 secondes après chaque addition unique,
f) la poudre obtenue et mélangée est tamisée avec un tamis de 140 mesh.

5. Procédé selon la revendication précédente, **caractérisé en ce que** :
a) 500 mg de poudre de tadalafil et 300 g d'une silice mésoporeuse granulaire, telle que l'aeroperl, sont placés à l'intérieur du flacon rotavapor ;
b) une quantité de solvant égale à 600 ml d'éthanol est ajoutée dans le même flacon ;
c) la solution contenant les poudres et le solvant est soniquée pendant 240 secondes ;
d) le flacon rotatif est relié au rotavapor pendant 40 minutes à 40°C afin de faire évaporer le solvant et d'obtenir l'échantillon d'aeroperl 300 imprégné de tadalafil ;
e) l'huile de serenoa est ensuite incorporée au moyen d'un moulin à couteaux, en ajoutant l'huile à 5 ml par étape, en faisant fonctionner le moulin à couteaux pendant 20 secondes après chaque ajout unique ;
f) la poudre obtenue et mélangée est tamisée avec un tamis de 140 mesh.

6. Procédé selon la revendication précédente, **caractérisé en ce que** le volume apparent de la poudre obtenue est de 95 ml, convenant ainsi à la préparation de 100 capsules de type « double zéro ».

7. Procédé selon la revendication précédente, **caractérisé en ce que** chaque capsule ou comprimé contient 5 mg de tadalafil et 200 mg d'extrait lipido-stérolique à 85-95 % de serenoa repens.
